# EUROPEAN PATENT SPECIFICATION

(11) **EP 4 099 858 B1**
(45) Date of publication and mention of the grant of the patent: **15.04.2026**
(21) Application number: 21703754.8
(22) Date of filing: 28.01.2021
(51) Int. Cl.: A24F 40/53, A61M 15/06, A24F 40/42

(54) **AEROSOL PROVISION SYSTEM**
AEROSOLBEREITSTELLUNGSSYSTEM
SYSTÈME DE FOURNITURE D'AÉROSOL

(30) Priority: 07.02.2020 GB 202001674
(43) Date of publication of application: 14.12.2022
(73) Proprietor: Nicoventures Trading Limited, London WC2R 3LA (GB)
(72) Inventor: CHEN, Shixiang, London Greater London WC2R 3LA (GB); MOLONEY, Patrick, London Greater London WC2R 3LA (GB)
(74) Representative: Whiting, Gary
(86) International application number: PCT/GB2021/050201
(87) International publication number: WO 2021/156604

(56) References cited:
- EP-A1- 2 399 636
- WO-A1-2016/005602
- WO-A1-2019/104227
- US-A1- 2019 289 915

## Description

### Technical Field

The present invention relates to an aerosol provision system, a cartridge, a control unit, a method of providing an aerosol, and aerosol provision means.

### Background

Aerosol provision systems (also known as aerosol provision devices) are known. Common devices use heaters to create an aerosol from an aerosol-generating material which is then inhaled by a user. Modem devices may be compatible with an array of aerosol-generating material. However preferred heating conditions for producing aerosols from different aerosol-generating material may vary.

It is desirable for aerosol provision systems to provide heating profiles which are suitable for the aerosol-generating material present in the system during use. Therefore there is a requirement to provide an improved aerosol provision system to reliably provide suitable heating profiles.

The present invention is directed toward solving some of the above problems.

WO 2016/005602 discloses an electrically operated aerosol-generating system comprising an aerosol-generating device, and first and second removable aerosol-forming cartridges each comprising a resistive heater. The first removable aerosol-forming cartridge comprises a first aerosol-forming substrate requiring a first heating profile and the second removable aerosol-forming cartridge comprises a second aerosol-forming substrate requiring a second heating profile. The aerosol-generating device comprises a control unit arranged to detect whether the first or second aerosol-forming cartridge has been received within a cavity of the aerosol-generating device based upon the resistive load of the respective resistive heater.

WO 2019/104227 discloses vaporizers and vaporizer systems, which can include a device in communication with a vaporizer, can include one or more features related to control of functions and/or features of the vaporizer, identification of a cartridge and/or a vaporizable material in the cartridge, data exchange (either one-way or two-way) between a cartridge and a vaporizer with which the cartridge is engaged, and the like.

US 2019/289915 discloses a vaporizer device including a body having a mouthpiece, a power source, a regulator coupled to the power source, a cavity configured to receive a vaporizer cartridge, and one or more airflow pathways configured to interconnect with an airflow coupler associated with a vaporizer cartridge. The vaporizer device may include a sensor configured to detect a pressure change in the one or more airflow pathways between the vaporizer cartridge and the mouthpiece. The vaporizer device may include a controller configured to control the regulator, and a data interface coupled to the processor, the data interface being configured to contact a receiving portion of a vaporizer cartridge when the vaporizer cartridge is inserted into the cavity.

EP 2399636 discloses a liquid storage portion comprising an electrical component for distinguishing the storage portion from other liquid storage portions is disclosed. The liquid storage portion is configured for use in an aerosol generating system having means for determining an electrical characteristic of the electrical component and means for distinguishing the liquid storage portion from other liquid storage portions based on the determined electrical characteristic of the electrical component.

### Summary

Aspects of the invention are defined in the accompanying claims.

In accordance with some embodiments described herein, there is provided a cartridge identification system configured to identify a cartridge for containing aerosol-generating material, the system comprising: a cartridge receiving unit configured to receive and/or engage with a cartridge, the cartridge receiving unit having a first plurality of contacts for connecting to a cartridge having a second plurality of contacts, wherein the cartridge receiving unit identifies a cartridge type associated with the cartridge based on which of the first plurality of contacts are in contact with which of the second plurality of contacts when the cartridge is coupled to the cartridge receiving unit for use.

In accordance with some embodiments described herein, there is provided a cartridge for use with a cartridge identification system, the cartridge comprising a region for containing aerosol generating material comprising: a plurality of cartridge contacts for connecting to a cartridge receiving unit configured to receive and/or engage with a cartridge, the cartridge receiving unit having a plurality of cartridge receiving unit contacts, wherein a cartridge type associated with the cartridge is identifiable based on which of the plurality of cartridge contacts are in contact with which of the plurality of cartridge receiving unit contacts when the cartridge receiving unit is coupled to the cartridge for use.

In accordance with some embodiments described herein, there is provided a cartridge receiving unit configured to receive and/or engage with a cartridge, comprising: a plurality of cartridge receiving unit contacts for connecting to a cartridge having a plurality of cartridge contacts, wherein the cartridge receiving unit identifies a cartridge type associated with the cartridge based on which of the plurality of cartridge receiving unit contacts are in contact with which of the plurality of cartridge contacts when the cartridge is coupled to the cartridge receiving unit for use.

In accordance with some embodiments described herein, there is provided a method of identifying a cartridge for containing an aerosol generating material for generating aerosol from an aerosol generating material, the method comprising: connecting at least two contacts of a first plurality of contacts of a cartridge receiving unit and a corresponding at least two contacts of a second plurality of contacts of a cartridge for an aerosol provision system; and identifying a cartridge type associated with the cartridge based on which of the first plurality of contacts are in contact with which of the second plurality of contacts.

In accordance with some embodiments described herein, there is provided a method of providing an aerosol using an aerosol provision system comprising a cartridge receiving unit with a first plurality of contacts and a cartridge with a second plurality of contacts, the method comprising: connecting at least two contacts of the first plurality of contacts of the cartridge receiving unit and a corresponding at least two contacts of the second plurality of contacts of the cartridge; identifying a cartridge type associated with the cartridge based on which of the first plurality of contacts are in contact with which of the second plurality of contacts; selecting a heating profile based on the identified cartridge type and, providing power to a heater in accordance with the heating profile to provide an aerosol from an aerosol-generating material.

In accordance with some embodiments described herein, there is provided a cartridge identification system configured to identify a cartridge for containing an aerosol generating material, the system comprising: cartridge receiving means having a first plurality of contact means for connecting to a cartridge having a second plurality of contact means, wherein the cartridge receiving means identifies a cartridge type associated with the cartridge based on which of the first plurality of contact means are in contact with which of the second plurality of contact means when the cartridge is coupled to the cartridge receiving means for use.

### Description of Drawings

The present teachings will now be described by way of example only with reference to the following figure:
Figure 1 is a perspective view of an aerosol provision system according to an example;
Figure 2 is a perspective view of an aerosol provision system according to an example;
Figure 3 is a longitudinal cross-sectional view of an aerosol provision system according to an example;
Figure 4 is a longitudinal cross-sectional view of an aerosol provision system according to an example;
Figure 5 is a longitudinal cross-sectional view of an aerosol provision system according to an example;
Figure 6 is a longitudinal cross-sectional view of an aerosol provision system according to an example; and,
Figure 7 is a longitudinal cross-sectional view of an aerosol provision system according to an example.

While the invention is susceptible to various modifications and alternative forms, specific embodiments are shown by way of example in the drawings and are herein described in detail. It should be understood, however, that the drawings and detailed description of the specific embodiments are not intended to limit the invention to the particular forms disclosed. On the contrary, the invention covers all modifications, equivalents and alternatives falling within the scope of the present invention as defined by the appended claims.

### Detailed Description

Aspects and features of certain examples and embodiments are discussed / described herein. Some aspects and features of certain examples and embodiments may be implemented conventionally and these are not discussed / described in detail in the interests of brevity. It will thus be appreciated that aspects and features of apparatus and methods discussed herein which are not described in detail may be implemented in accordance with any conventional techniques for implementing such aspects and features.

The present disclosure relates to aerosol provision systems, which may also be referred to as aerosol provision systems, such as e-cigarettes. Throughout the following description the term "e-cigarette" or "electronic cigarette" may sometimes be used, but it will be appreciated this term may be used interchangeably with aerosol provision system / device and electronic aerosol provision system / device. Furthermore, and as is common in the technical field, the terms "aerosol" and "vapour", and related terms such as "vaporise", "volatilise" and "aerosolise", may generally be used interchangeably.

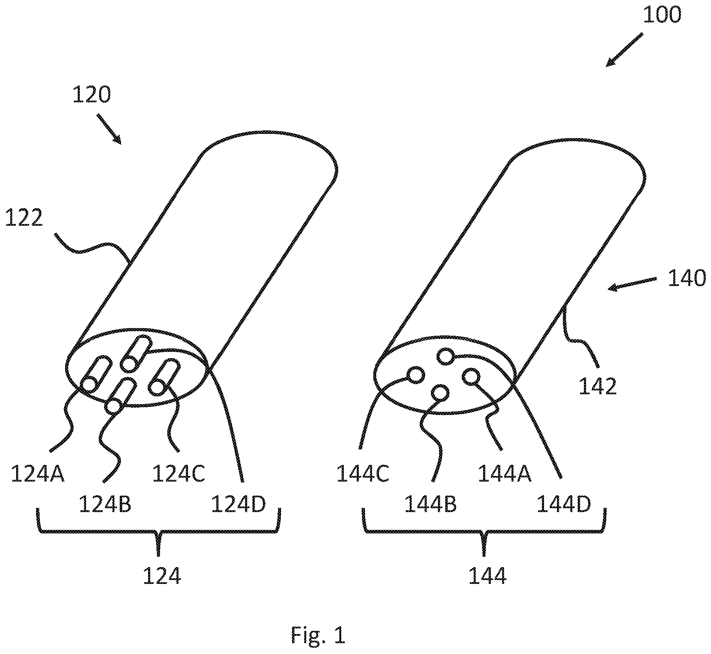

As shown in the example of Figure 1, a cartridge identification system 100 is disclosed which comprises a cartridge receiving unit 120 and a cartridge 140. In this example, the cartridge identification system 100 is an aerosol provision system 100 configured to generate aerosol from an aerosol-generating material provided in the cartridge 140. The cartridge receiving unit 120 may be a control unit 120 (or sometimes referred to as an aerosol provision device) configured to control the generation of aerosol (and may optionally control other functions of the aerosol provision system). The control unit 120 and cartridge 140 combined form the aerosol provision system 100.

As shown, the control unit 120 has a plurality of contacts 124A, 124B, 124C, 124D (124) for connecting to the cartridge 140 having a second plurality of contacts 144A, 144B, 144C, 144D (144). The control unit 120 identifies the cartridge 140 (e.g., by identifying a cartridge type associated with the cartridge 140) based on which of the first plurality of contacts 124 are electrically coupled with which of the second plurality of contacts 144 when the cartridge 140 is coupled to the control unit for use. For example, the control unit 140 may determine which ones of the first plurality of contacts 124 are in contact with corresponding ones of the second plurality of contacts 144, and the control unit 140 subsequently identifies the cartridge 140 based on the determination. "Connecting" as used herein in relation to the control unit 120 and the cartridge 140 refers to a physical and electrical connection between these components. As such, the contacts 124, 144 are electrical contacts. The contacts 124, 144 may be made of metal or other suitable electrically conductive material. The contacts 124 of the control unit 120 may be referred to as control unit contacts 124. The contacts 144 of the cartridge 140 may be referred to as cartridge contacts 144. It should be appreciated that in some implementations, the electrical connection may include an inductive or capacitive connection. That is, the contacts 124, 144 may not necessarily physically contact one another, but are provided such that when the cartridge 140 is physically connected to the control unit 120, the ones of the respective contacts 124, 144 are electrically connected.

The control unit 120 and the cartridge 140 of the aerosol provision system 100 are releasably connectable. Prior to use, the user may connect the control unit 120 to the cartridge 140. In connecting the control unit 120 to the cartridge 140, the contacts 124 of the control unit 120 are aligned with the respective contacts 144 of the cartridge 140. In the example of Figure 1, the control unit 120 has four contacts 124A, 124B, 124C, 124D and the cartridge 140 has four contacts 144A, 144B, 144C, 144D.

The control unit 120 shown in Figure 1 has a body 122. The body 122 has a cross section perpendicular to the elongate axis of the body 122 in the shape of an oval. In this example, the contacts 124 are arranged roughly in the shape of a diamond within the oval cross section. The cartridge 140 has a body 142. The body 142 of the cartridge 140 has a corresponding cross section to the control unit 120 and therefore has a cross section perpendicular to the elongate axis of the body 142 in the shape of an oval. In this example, the contacts 144 are also arranged roughly in the shape of a diamond within the oval cross section, again corresponding with the contact arrangement of the control unit 120. In other implementations, the contacts 124, 144 may be arranged in other shapes, wherein at least a part of the arrangement of shapes of contacts 124 and 144 are corresponding.

In connecting the control unit 120 to the cartridge 140:
the leftmost contact 124A of the control unit 120 may be connected to the rightmost contact 144A of the cartridge 140;
the lower contact 124B of the control unit 120 may be connected to the lower contact 144B of the cartridge 140;
the rightmost contact 124C of the control unit 120 may be connected to the leftmost contact 144C of the cartridge 140; and,
the upper contact 124D of the control unit 120 may be connected to the upper contact 144D of the cartridge 140.

In this way the control unit 120 may be connected to the cartridge 140.

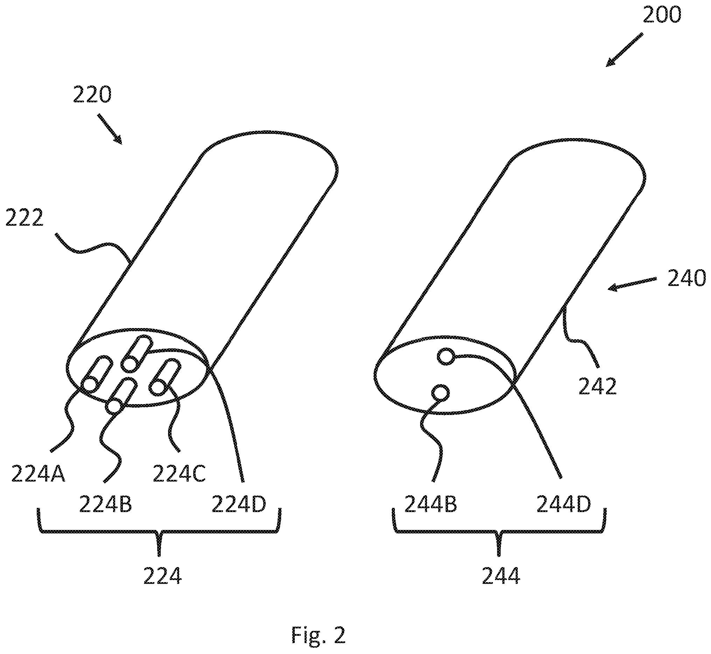

In Figure 2, an example of an aerosol provision system 200 is shown. The system 200 shown in Figure 2 has a number of similar features to the system 100 shown in Figure 1. Numerals referring to similar features from Figure 1 have had their number increased by 100. Similar features may not be discussed in detail here for efficiency.

The cartridge 240 in Figure 2 has two contacts 244B, 244D (244) arranged in the upper and lower positions as shown in Figure 1. Figure 2 does not show the cartridge 240 as having contacts in the leftmost and rightmost positions, as shown in Figure 1. These contacts may be omitted or may be covered up by e.g. a non-electrically conductive material. Prior to use of the system 200, the user may connect the control unit 220 to the cartridge 240. The upper and lower contacts 224B, 224D of the control unit 220 connect to the upper and lower contacts 224B, 224D of the cartridge 240.

In both of the arrangements described in Figures 1 and 2, the control unit 120, 220 is configured to determine which of the contacts 124, 224 on the control unit 120, 220 are electrically coupled to respective ones of the contacts 144, 244 of the cartridge 140, 240.

For example, at least one of the contacts 124, 224 is, or can be set as, a power input; that is, power is applied to the contact 124 from a power source, such as a battery or the like, provided in the control unit 120, 220. When the power input contact 124, 224 is electrically coupled to a contact 144, 244 on the cartridge, which itself is electrically coupled to another of the contacts 144, 244 on the cartridge 140, 240 and to one of the remaining contacts 124, 224 on the control unit 120, 220, then a current is permitted to flow from the power input contact 124, 224 thought the cartridge 140, 240 and back to the other of the contacts 124, 224.

The control unit 120, 220 is configured to determine for various ones of contacts 124, 224 whether or not a current is flowing (e.g., by measuring a current, resistance, or voltage). This may be achieved using a suitable sensor positioned in the control unit (such as a current sensor). As should be appreciated, the way in which the contacts 144, 244 of one cartridge 140, 240 are configured as compared to the contacts 144, 244 of another cartridge 140, 240 may differ, and accordingly the control unit 120, 220 can determine the configuration of the contacts 144, 244 of the cartridge 140, 240 based on which contacts 124, 224 are determined to have a current flowing.

In some implementations, at least one pair of contacts 144, 244 in the cartridge 140, 240 is provided, where "a pair of contacts" should be understood to mean two contacts that are electrically coupled together within the cartridge 140, 240. That is, a first contact 144, 244 is electrically coupled to a second contact 144. 244 within the cartridge 140, 240, e.g., via an electrically conductive element such as a wire. As described above, when power is applied to one of the pair of contacts, an electrical circuit can be completed with respective ones of the contacts 124, 224 of the control unit 120, 240. The control unit 120, 220 is arranged to determine the configuration of the contacts 144, 244 of the cartridge 140, 240 based on which pair (or pair) of contacts 124, 224 are determined to have a current flowing. That is, for a set of cartridges 140, 240 having a certain number of contacts 144, 244, different combinations of paired contacts (and/or unpaired contacts) can be attributed to different cartridge types (e.g., the flavour of liquid stored within the cartridge).

In more detail, the control unit 120, 220 may have a power source such as a battery or the like. The control unit 120, 220 may have a controller for controlling delivery of power from the power source. In particular, the controller may control current being delivered from the power source to the contacts 124, 224 of the control unit 220. When the control unit 220, as shown in Figure 2, of the aerosol provision system 200 is connected to the cartridge 240, the controller may selectively direct current to the contacts 224. The controller can selectively provide power to, for example, leftmost and rightmost contacts 224A, 224C. These may be the outward and return signal contacts respectively, or vice versa. The controller can detect that there is no current passing between these contacts 224A, 224C - this is as a result of the contacts 224A, 224C not being electrically connected to cartridge 240 (e.g., because contacts 244B, 244D on the cartridge 240 do not align with contacts 224A, and 224C) and therefore a complete circuit is not provided. The controller can then stop providing power to the leftmost and rightmost contacts 224A, 224C.

The controller may then selectively provide power to the upper and lower contacts 224B, 224D of the control unit 220. When the control unit 220 of Figure 2 is connected to the cartridge 240, the upper and lower contacts 224B, 224D of the control unit 220 are in electrical contact with the contacts 244B, 244D of the cartridge 240. As such, there is a complete circuit from the control unit 220 to the cartridge 240 and back, where it is understood that contacts 244B and 244D are electrically connected to one another within the cartridge body 240. The controller can direct power through these contacts 224B, 224D and detect that there is a current passing through contacts 224B, 224D, 244B, 244D, from the power source of the control unit 220 to the cartridge 240 and back. For example, the control unit 220 may include a current sensor (not shown), which may form part of the controller. The controller may also or alternatively measure a voltage between the contacts 224B, 224D. A predefined voltage will only exist, and therefore be detectable, if a circuit has been formed from the control unit 220 to the cartridge 240 and back.

The cartridge 240 may have a vaporiser (which in this example is a heater) for vaporising an aerosol-generating material and aerosol-generating material. The power supplied from the control unit 220 may be provided to the heater to provide thermal energy to the aerosol-generating material so as to generate an aerosol from the aerosol-generating material. The heater may be a resistive heater, and inductive heater, an optical/infrared heater, for example. In other implementations, the vaporiser may be a vibrating aerosoliser such as a piezoelectric mesh dish.

In the manner as described above, the control unit 120, 220 may be able to detect which contacts of the control unit 120, 220 are electrically connected to the cartridge 240. This gives an indication of the spatial and/or electrical arrangement of the contacts 244 on the cartridge 240. The arrangement of contacts 244 on the cartridge 240 can therefore be used to identify the type of the cartridge 240, such that cartridges 240 with different arrangements of contacts 240 can be distinguished between. For instance, cartridge 240 in Figure 2 can be distinguished from cartridge 140 in Figure 1 based on the detected current. The cartridge type may be directly related to the arrangement of contacts 244 on the cartridge. If the arrangement of contacts 244 is further related to the aerosol-generating material stored within the cartridge, the aerosol-generating material in the cartridge 240 may be identified by the arrangement of contacts 244 on the cartridge 240. The term "cartridge type" as used herein should be understood to encompass properties of the cartridge, which may include at least one of: a certain construction of the cartridge (e.g., a certain physical shape of the cartridge, or a part thereof e.g., reservoir volume), an aerosol generating material stored in the cartridge (e.g., a type or constituent), or a component of the cartridge (e.g., the heater/vaporiser used).

Therefore the manufacturer can produce cartridges with select contact arrangements which the controller is pre-programmed to recognise as certain cartridge types, e.g., containing specific aerosol-generating material. The controller may at that point automatically set, or provide an option for a user to manually set, a predetermined heating profile from the heater to the aerosol-generating material in the cartridge, to ensure that a suitable aerosol is provided from the system 100, 200 when the system 100, 200 is activated. In this way, the control unit 120, 220 of the system 100, 200 can be used with a plurality of different cartridges 140, 240 containing a plurality of different aerosol-generating material and provide a suitable aerosol from each by supplying a suitable level of power/heating without requiring any input from the user. Therefore, the different aerosol-generating material may be heated using a heating profile that is suitable for that aerosol-generating material (i.e. the power supplied is great enough), but that the different aerosol-generating material and/or device components are not damaged (e.g. burnt, deformed or degraded) due to excessive power. Furthermore, the user experience of the system 100, 200 is significantly improved and the reusability of the control unit 120, 220 means the lifetime of the system 100, 200 is significantly improved.

The cartridge 140, 240 may have an outlet or mouthpiece or the like, for allowing aerosol to pass from the cartridge 140, 240 to the outside of the system 100, 200. Multiple users may therefore share a single control unit 120, 220 while having their own personal cartridge 140, 240. As the parts of the system 100, 200 are easy to connect, the user simply disconnects one cartridge 140, 240 from the control unit 120, 220 and then connects another cartridge 140, 240. This provides the user with an easy method of switching between aerosol-generating material without requiring a plurality of devices with which to do so and without needing to alter the heating profile provided by the control unit 120, 220.

In this way, the multi-pin arrangement of the control unit 120, 220 as shown herein is used to enable the control unit 120, 220 to recognise the type of cartridge 140, 240 in use and therefore accommodate the aerosol-generating material appropriately. This, in turn, may assist in reducing the effects of hot puff and dry out as an appropriate heating profile will be delivered to the aerosol-generating material contained in the cartridge 140, 240. The heating profile delivered may take into account any of the following: different forms of aerosol-generating material (e.g. solid, gel or liquid); different flavours of aerosol-generating material; different aerosolisation temperatures; different notes within the aerosol to emphasise and the heating profile required to do so; quantity of aerosol-generating material and therefore how many times the cartridge may be used prior to a replacement being needed; different heater configurations (e.g. resistive heater(s), or inductive heater(s)).

In a specific example, the control unit 120, 220 may have four contacts 124, 224. The contacts 124, 224 may have two outward signal contacts 124A, 224A, 124B, 224B and two return signal contacts 124C, 224C, 124D, 224D. In this example, combinations of the outward and return contacts 124, 224 can be used to identify the cartridge 140, 240 in use. In an arrangement with more contacts, more combinations of contact pairs are provided which enables a greater number of cartridges to have differentially identifiable contact arrangements.

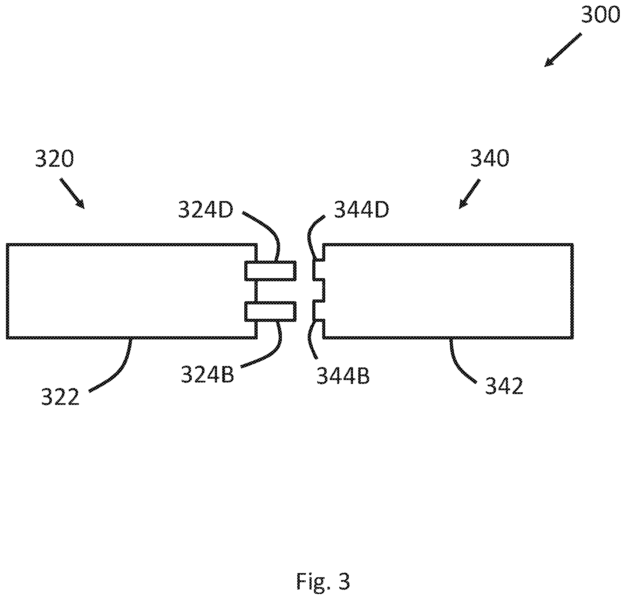

In Figure 3, a longitudinal cross section of an example of an aerosol provision system 300 is shown. The system 300 shown in Figure 3 has a number of similar features to the system 100, 200 shown in Figures 1 and 2. Numerals referring to similar features from Figure 2 have had their number increased by 100. Similar features may not be discussed in detail here for efficiency.

The contacts 324B, 324D of the control unit 320 are shown as projecting outwardly from the main body 322 of the control unit 320. The contacts 344B, 344D of the cartridge 340 are also shown as projecting outwardly from the main body 342 of the cartridge 340 - alternatively, the contacts may be flat contact points on the cartridge 340. This projected position of the contacts 324, 344 is the at rest position of the contacts 324, 344. In connecting the control unit 320 and the cartridge 340, the user may push the contacts 324B, 324D, 344B, 344D together. The contacts 324B, 324D, 344B, 344D may be somewhat retractable or set in a resilient material to enable partial retraction. This partial retraction may occur when the contacts 324, 344 encounter a force against them. This arrangement allows for relative movement between the contacts 324B, 324D. 344B, 344D to assist the contacts 324B, 324D, 344B, 344D in not breaking when pushed together. This arrangement also assists the contacts 324B, 324D, 344B, 344D in being held together in contact while also assisting the body 322 of the control unit 320 and the body 342 of the cartridge 340 to abut. Abutting of the bodies 322, 342 further assists in providing a reliable electrical connection between the contacts 324B, 324D, 344B, 344D.

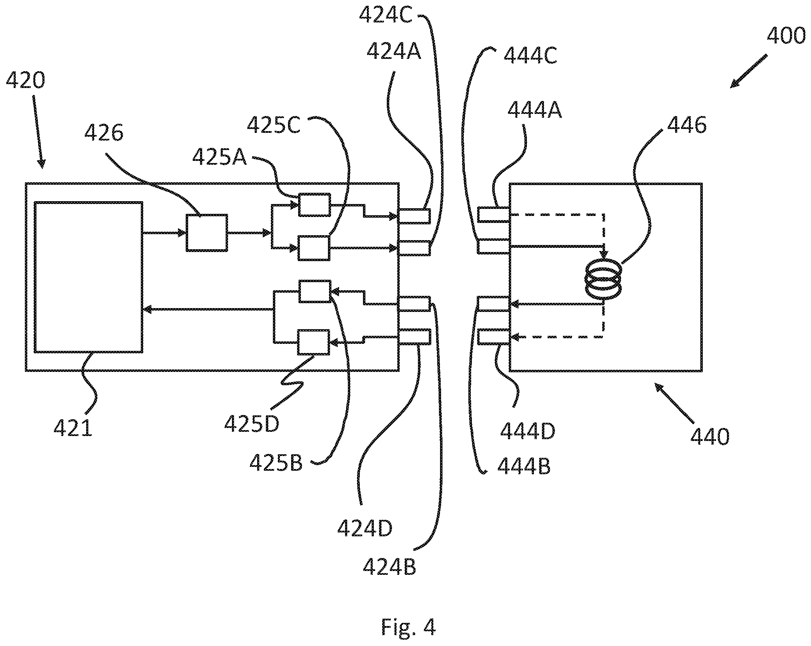

In Figure 4, a longitudinal cross section of an example of an aerosol provision system 400 is shown. The system 400 shown in Figure 4 has a number of similar features to the systems 100, 200, 300 shown in Figures 1 to 3. Numerals referring to similar features from Figure 1, Figure 2 and Figure 3 have had their number increased by 300, 200 and 100 respectively. Similar features may not be discussed in detail here for efficiency.

The control unit 420 has a power source 421 such as a battery or the like. The control unit 420 also has contacts 424A, 424B, 424C, 424D as previously described. The control unit 420 shown has switches 425A, 425B, 425C, 425D to control the delivery of power to the contacts 424. The control unit 420 has positive switches 425A, 425C for controlling the outward path of power delivery to contacts 424A, 424C respectively. The control unit 420 has negative switches 425B, 425D for controlling the return path of power delivery from contacts 424B, 424D respectively. The control unit 420 has a current sensor 426 for detecting passage of current from the power source 421. This, in use, can confirm whether the power delivery from the power source 421 to the cartridge 440 is successfully moving through a complete circuit.

The cartridge 440 has contacts 444A, 444B, 444C, 444D as previously described. The cartridge 440 has a heating element 446, which in this example is a coil formed of a resistive wire (e.g., NiChrome). There is circuitry shown in Figure 4 from the positive (outward) contacts 444A, 444C to the heating element 446, but the connection between either of the two contacts 444A, 444C to the heating element 446 may or may not exist. There is circuitry shown in Figure 4 from the heating element 446 to the negative (return) contacts 444B, 444D, but the connection between either of the two contacts 444B, 444D to the heating element 446 may or may not exist.

The configuration shown in Figure 4 has a 4-contact point system, though the number of contacts may vary. To deliver power to the heating element 446, a complete circuit from the power source 421, to the heating element 446 and back via any combination of contacts 424A, 424B, 424C, 424D, 444A, 444B, 444C, 444D needs to exist. In a minimal contact arrangement, a corresponding pair of the outward contacts 424A, 424C, 444A, 444C are active and the same for the return contacts 424B, 424D, 444B, 444D. In a specific example this could be the contacts 424A, 444A and contacts 424B, 444B. In a maximal contact arrangement, all contacts 424, 444 may be used, with current being shared between the two parallel contacts. The contacts are in a parallel circuit arrangement to enable selective activation of the paths corresponding to the contacts to occur.

As can be appreciated, there are a number of paths that can be formed using the 4 contact pairs. Each of these paths can relate to a specific cartridge 440 which enables the control unit 420 to identify the cartridge 440 in use. Indeed with the contact arrangement shown in Figure 4, there are nine options in relation to the cartridge 440:
Option 1: Contacts 444A and 444B are used;
Option 2: Contact 444A and 444D are used;
Option 3: Contact 444C and 444B are used;
Option 4: Contact 444C and 444D are used;
Option 5: Contact 444A, 444C and 444B are used;
Option 6: Contact 444A, 444C and 444D are used;
Option 7: Contact 444A, 444B and 444D are used;
Option 8: Contact 444C, 444B and 444D are used; and,
Option 9: Contact 444A, 444C, 444B and 444D are used.

As mentioned above, the 4-point contact system shown in Figure 4 could instead be a 6-contact point system with 3 positive contacts points and 3 negative contact points which leads to 49 different arrangements of the 6 contact points on the cartridge 440. This therefore can cover a large number of cartridge types and therefore associated aerosol-generating material. Of course, 5 point contact systems with 3 positive contact points and 2 contact negative points (or vice versa) may be used. Indeed a 3 point contact system could be used, although the possible combinations decrease significantly.

In the example of Figure 4, the control unit 420 sends a test signal through all 4 possible one to one connection combinations of contacts 424A, 424C and 424B, 424D using the current sensor 426 and the 4 switches 425A, 425B, 425C, 425D (located between the current sensor 426 and the contacts 424,) for the connection to the 4 possible contact points 444A, 444B, 444C, 444D on the cartridge 440. Therefore, so long as a specific type of cartridge 440 exclusively uses one of the 9 options of contact point arrangement for power delivery, once such an arrangement is identified by the system 400, the cartridge type is hence identified.

The identification of the connection arrangement of a given cartridge 440 can be achieved after completing the following 4 tests: -
Test 1: Send a test signal when only Switch 425A and 425B are turned on;
Test 2: Send a test signal when only Switch 425A and 425D are turned on;
Test 3: Send a test signal when only Switch 425C and 425B are turned on; and
Test 4: Send a test signal when only Switch 425C and 425D are turned on.

Based on the current reading at the current sensor 426 in these tests, the control unit 420 can identify which of these 4 tests can successfully send an electrical current (a test signal) through a given cartridge 440 with the defined connection arrangement. Therefore, it can identify the exact Option among the 9 contact arrangement options used by this cartridge according to the following rules:
If only Test 1 is successful, the given cartridge is using Option 1;
If only Test 2 is successful, the given cartridge is using Option 2;
If only Test 3 is successful, the given cartridge is using Option 3;
If only Test 4 is successful, the given cartridge is using Option 4;
If only Test 1 and Test 3 are successful, the given cartridge is using Option 5;
If only Test 2 and Test 4 are successful, the given cartridge is using Option 6;
If only Test 1 and Test 2 are successful, the given cartridge is using Option 7;
If only Test 3 and Test 4 are successful, the given cartridge is using Option 8; and
If any three or all of the 4 tests are successful, the given cartridge is using Option 9.

In practice, cartridges 440 may be manufactured with different contact point arrangements. It can be achieved by having all possible contact points built on the cartridge 440 but covering those unwanted ones with electrical insulation material, such as a plastic cover. To achieve better security, for a given type of cartridge 440, it can also be built only with the contact points for a selected arrangement.

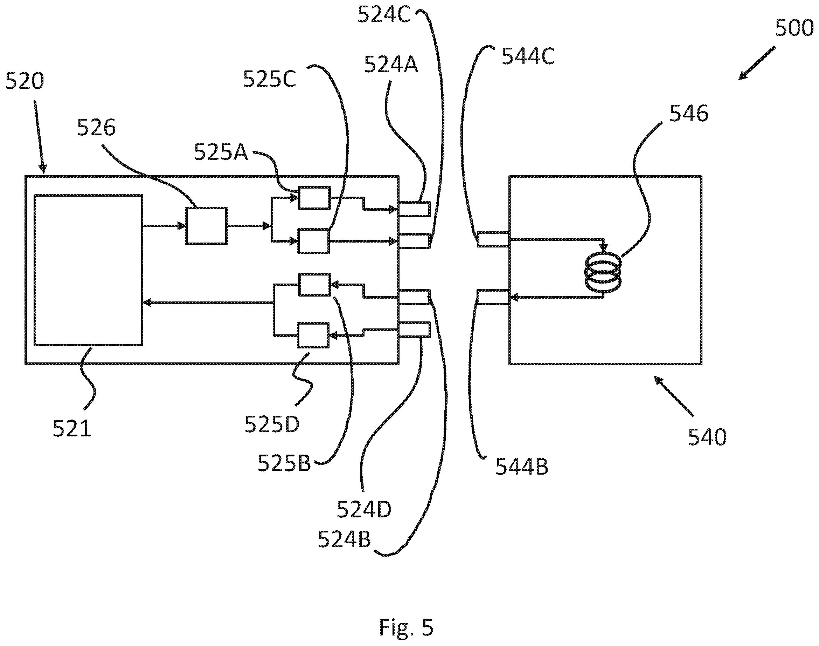

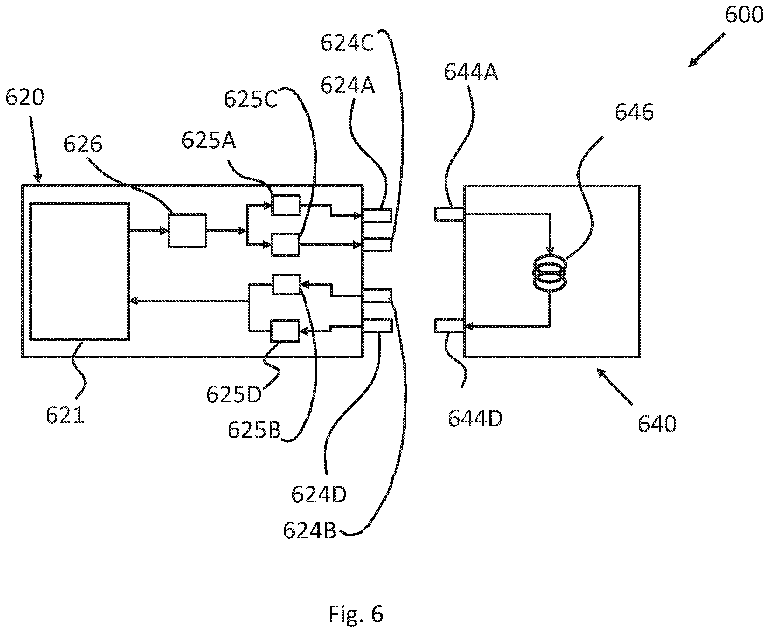

Figures 5 and 6 show embodiments wherein the specific contact combinations are used. In particular, Figure 5 illustrates an example where contacts 524C, 544C, 524B, 544B (Option 3) are used. Figure 6 illustrates and example where contacts 624A, 644A, 624D, 644D (Option 2) are used.

In Figures 4 to 6, there may be one input and one output on the cartridge 440, 540, 640, or there may be multiple inputs and one output or one input and multiple outputs. In use, the controller may detect which cartridge 440, 540, 640 is attached by detecting which contacts are active and/or where those contacts are positioned. Different cartridges 440, 540, 640 may have different contacts 444, 544, 644 arrangements or different input/output arrangements. The controller can then deliver a suitable heating profile based on the contents of the cartridge 440, 540, 640 (in terms of the aerosol generating material within the cartridge and/or the heater (or more generally aerosol generator) type). E.g. in Figure 4, all contacts in the cartridge 440 can be coupled in pairs (e.g., 444A electrically coupled to 444D, and 444C electrically coupled to 444D) or the contacts can be isolated (e.g., 444A may not be electrically coupled to any other contact in the cartridge).

In Figure 4, contact 444C is shown as connected to 444B. Contact 444C may also or alternatively be connected to 444D, in some instances. Therefore, when an input current is applied to contact 444C, a reading may be detected on both 424B and 424D. The processor may be configured to determine a first cartridge (or first cartridge type) if 424B is positive (and 424D negative), and a second cartridge if 424D is positive (and 424B is negative), and a third cartridge if 424B and 424D are positive. That is, in some implementations, a given contact on the cartridge may be electrically coupled to at least one other contact on the cartridge. This arrangement therefore increases the number of available combinations as a result of enabling the ability to distinguish between more types of cartridge without having to increase the number of contacts in the system 400.

The heaters 446, 546 shown in Figures 4 and 5 are schematically shown as between the paired contacts. In an exemplary arrangement, the heater may be arranged between only two contacts and not connected to any other contacts. This is particularly the case where electrical power is to be supplied by a direct electrical and physical connection to the heater (that is, in system where the heating mechanism is resistive). Thus, in some implementations, there will be at least one pair of contacts of the cartridge that are coupled together with this pair of contacts electrically coupled to the heater (e.g., physically connected to either side of the heater). In an example, this may provide a separate set of contacts on the device (i.e., contacts not connected to a heater). In some implementations, this may result in a cartridge having a dedicated set of contacts for supplying power to the heater (which may be the same pair of contacts for every cartridge) and a separate set of contacts which may operate as an identification set of contacts in accordance with the principles described above. Thus, broadly speaking, the present disclosure may be said to provide a plurality of electrical contacts on the cartridge, whereby an electrical contact on the cartridge may be said to have "a heater power supply function", "an identification function" or both, depending on the arrangement of the system.

In such implementations, electrical power sufficient to cause heating of the heater to generate aerosol from the aerosol generating material may be supplied on the set of contacts having a heater power supply function, whereas a different electrical power (e.g., a lower magnitude) may be supplied to the contacts having an identification function. As described above, the set of identification contacts may be coupled together in any suitable combination or isolated from one another. Accordingly, it should be understood that, in terms of electrical contacts regardless of function, implementations of the present disclosure provide cartridges with at least one pair of contacts electrically coupled together and electrically coupled to the heater, and at least one other contact (which may or may not be electrically coupled to another contact of the cartridge). In other implementations, such as where the heater is inductively heated and a physical connection to the heater (or more generally aerosol generator) is not required, the cartridge may comprise at least one electrical contact (which may or may not be electrically coupled to another contact of the cartridge). It should be appreciated that such inductive heating arrangements do not preclude the heater (susceptor) being electrically connected to one or more identification contacts.

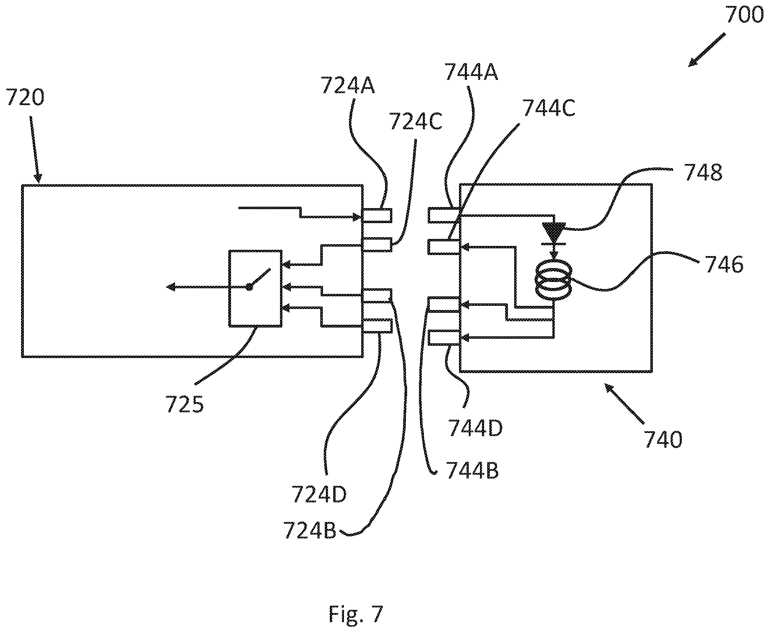

In an example, shown in Figure 7, the contacts 724A, 744A control the output from the control unit 720 and the input to the cartridge 740. This passes through the heater 746 and then may be passed to one or more of contacts 744B, 744C, 744D. These contacts 744B, 744C, 744D connect to the control unit 720 and may be fed to a switch 725 which may alternate between the contacts 724B, 724C, 724D. Each contact 724B, 724C, 724D may be tested to detect the feed (i.e., whether a current is detected on one or more of contacts 724B, 724C, 724D). Accordingly, a controller (not shown) may be used to identify the cartridge 740 based on the detected feed from contacts 724B, 724C, 724D. As before, the cartridge 740 contents may vary depending on which of contacts 744B, 744C, 744D is coupled to the input contact 744A, and a suitable action may be taken based on the determined identity of the cartridge (e.g., such as applying a suitable heating profile). In this example, the outputs of the cartridge are switched between. In a similar manner, in an alternative arrangement, the inputs may be switched with a slightly amended arrangement. It should be appreciated that any combination of the above techniques may be utilised in a particular system.

In some examples, diodes may be located between one or more pairs of contacts to ensure power travels in specific directions. E.g. from one specific contact to another, or in a specific direction. In this regard, supposing contact 744A is electrically coupled to contact 744B. Without a diode, applying power to contact 744A and detecting the output at contact 744B provides the same result as applying power to contact 744B and detecting the output at 744A as power is free to flow in either direction. By inserting a diode, this adds another factor which can alter the number of possible combinations by which the contacts can be coupled. In other words, adding a diode may increase the number of identifiable combinations (and thus identifiable types of cartridges) for a given set of electrical contacts. In the example of Figure 7, there is a diode 748 located between the contact 744A and the heating element 746. As mentioned above, a diode may vary in its use, location and diode direction in different examples of any of the above systems.

In other example systems, the cartridge may be for use with an aerosol provision device but not necessarily in the aerosol provision device or system. In an example, the cartridge may be a container of bulk liquid which can be recognised by a control unit (via the electrical connections as described above) as containing a specific bulk liquid. The control unit may then utilise this information by e.g. informing an aerosol provision device or system to use a specific heating profile. In an example, this "informing" may be by any of wireless or wired signalling or the like.

While the above described implementations have focused on the cartridge identification system 100 being an aerosol provision system 100 configured to generate aerosol from an aerosol-generating material provided in the cartridge 140, the principles of the present disclosure may more generally be applied to other cartridge identification systems 100. That is, the cartridge receiving unit 120 may not necessarily be configured to generate aerosol from the cartridge 140 but may nevertheless be configured to recognise the cartridge 140. i That is, once identified, the cartridge receiving unit may perform a suitable action (depending on the nature of the cartridge receiving unit) on the basis of the identified cartridge, e.g., which may include but is not limited to setting an appropriate heating profile for the aerosol-forming material in the cartridge or the like.

The aerosol-generating material in the cartridge 140, 240, 340 of the system 100, 200, 300 may comprise at least one of tobacco and glycol and may include extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamon, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. The different aerosol-generating material may be separated, adjacent or overlapping.

Aerosolisable material, which also may be referred to herein as aerosol generating material, is material that is capable of generating aerosol, for example when heated, irradiated or energized in any other way. Aerosolisable material may, for example, be in the form of a solid, liquid or gel which may or may not contain nicotine and/or flavourants. The aerosol-generating material described herein may comprise an "amorphous solid", which may alternatively be referred to as a "monolithic solid" (i.e. non-fibrous), or as a "dried gel". The amorphous solid is a solid material that may retain some fluid, such as liquid, within it. In some cases, the aerosol-generating material comprises from about 50wt%, 60wt% or 70wt% of amorphous solid, to about 90wt%, 95wt% or 100wt% of amorphous solid. In some cases, the aerosol-generating material consists of amorphous solid.

The aerosol forming material may comprise one or more of glycerine, glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

The aerosolisable material may comprise an active material, an aerosol forming material and optionally one or more functional materials.

The active material may comprise nicotine or one or more other non-olfactory physiologically active materials. A non-olfactory physiologically active material is a material which is included in the aerosolisable material in order to achieve a physiological response other than olfactory perception.

The one or more functional materials may comprise one or more of flavours, carriers, pH regulators, stabilizers, and/or antioxidants.

The aerosolisable material may be present on a substrate. The substrate may, for example, be or comprise paper, card, paperboard, cardboard, reconstituted aerosolisable material, a plastics material, a ceramic material, a composite material, glass, a metal, or a metal alloy.

Thus there has been described a system configured to identify a cartridge, the system comprising: a cartridge receiving unit configured to receive and/or engage with a cartridge, the cartridge receiving unit having a first plurality of contacts for connecting to a cartridge having a second plurality of contacts, wherein the cartridge receiving unit identifies a cartridge type associated with the cartridge based on which of the first plurality of contacts are in contact with which of the second plurality of contacts when the cartridge is coupled to the cartridge receiving unit for use.

The aerosol provision system may be used in a tobacco industry product, for example a non-combustible aerosol provision system.

In one embodiment, the tobacco industry product comprises one or more components of a non-combustible aerosol provision system, such as a heater and an aerosolizable substrate.

In one embodiment, the aerosol provision system is an electronic cigarette also known as a vaping device.

In one embodiment the electronic cigarette comprises a heater, a power supply capable of supplying power to the heater, an aerosolizable substrate such as a liquid or gel, a housing and optionally a mouthpiece.

In one embodiment the aerosolizable substrate is contained in or on a substrate container. In one embodiment the substrate container is combined with or comprises the heater.

In one embodiment, the tobacco industry product is a heating product which releases one or more compounds by heating, but not burning, a substrate material. The substrate material is an aerosolizable material which may be for example tobacco or other non-tobacco products, which may or may not contain nicotine. In one embodiment, the heating device product is a tobacco heating product.

In one embodiment, the heating product is an electronic device.

In one embodiment, the tobacco heating product comprises a heater, a power supply capable of supplying power to the heater, an aerosolizable substrate such as a solid or gel material.

In one embodiment the heating product is a non-electronic article.

In one embodiment the heating product comprises an aerosolizable substrate such as a solid or gel material, and a heat source which is capable of supplying heat energy to the aerosolizable substrate without any electronic means, such as by burning a combustion material, such as charcoal.

In one embodiment the heating product also comprises a filter capable of filtering the aerosol generated by heating the aerosolizable substrate.

In some embodiments the aerosolizable substrate material may comprise an aerosol or aerosol generating agent or a humectant, such as glycerol, propylene glycol, triacetin or diethylene glycol.

In one embodiment, the tobacco industry product is a hybrid system to generate aerosol by heating, but not burning, a combination of substrate materials. The substrate materials may comprise for example solid, liquid or gel which may or may not contain nicotine. In one embodiment, the hybrid system comprises a liquid or gel substrate and a solid substrate. The solid substrate may be for example tobacco or other non-tobacco products, which may or may not contain nicotine. In one embodiment, the hybrid system comprises a liquid or gel substrate and tobacco. For example, the hybrid system may comprise a liquid or gel containing cartridge which generates an aerosol from the liquid or gel at an aerosol generating region of the cartridge (e.g., by applying sufficient heat to the liquid or gel), with a solid material (such as material comprising or consisting of tobacco) positioned downstream of the aerosol generating region such that the aerosol passes through (or around) the solid material.

In order to address various issues and advance the art, the entirety of this disclosure shows by way of illustration various embodiments in which the claimed invention(s) may be practiced and provide for a superior electronic aerosol provision system. The advantages and features of the disclosure are of a representative sample of embodiments only, and are not exhaustive and/or exclusive. They are presented only to assist in understanding and teach the claimed features. It is to be understood that advantages, embodiments, examples, functions, features, structures, and/or other aspects of the disclosure are not to be considered limitations on the disclosure as defined by the claims or limitations on equivalents to the claims, and that other embodiments may be utilised and modifications may be made without departing from the scope of the disclosure.

In addition, the disclosure includes other inventions not presently claimed, but which may be claimed in future.

## Claims

1. A cartridge identification system (100, 200...,700) configured to identify a cartridge (140, 220..., 720) for containing an aerosol-generating material, the system comprising:
a cartridge (140, 220 ..., 720) comprising a region for containing aerosol-generating material and having a plurality of cartridge contacts (144, 244..., 744);
a control unit (120, 220..., 720) configured to receive and/or engage with the cartridge (140, 220..., 720), the control unit having a plurality of control unit contacts (124, 224..., 724) for connecting to the cartridge (140, 220..., 720),
wherein at least one of the plurality of cartridge contacts (144, 244..., 744) is electrically coupled to at least one other of the plurality of cartridge contacts (144, 244..., 744) within the cartridge to form at least one pair of contacts,
wherein the plurality of control unit contacts comprises at least four contacts, and wherein at least two of the plurality of control unit contacts (124, 224..., 724) are power inputs,
the control unit (120, 220..., 720) further comprising:
a power source (421); and
a controller for controlling delivery of power from the power source;
wherein the controller is configured to selectively provide power to the at least two power inputs of the plurality of control unit contacts (124, 224..., 724), and the control unit (120, 220..., 720) identifies a cartridge type associated with the cartridge (140, 220..., 720) based on which of the plurality of control unit contacts (124, 224..., 724) are in contact with which of the plurality of cartridge contacts (144, 244..., 744) and which of the at least one pair of contacts are determined to have a current flowing when the cartridge (140, 220..., 720) is coupled to the control unit (120, 220..., 720) for use.

2. A system (100, 200...,700) according to claim 1, wherein the control unit (120, 220..., 720) is arranged to be releasably coupled to the cartridge (140, 220..., 720) in use.

3. A system (100, 200...,700) according to any of claims 1 to 2, wherein the controller controls delivery of power from the power source for operating a heater (446, 546, 746), and
wherein power from the power source is delivered to the cartridge (140, 220..., 720) via the contacts of the plurality of control unit contacts (124, 224..., 724) in contact with contacts of the plurality of cartridge contacts (144, 244..., 744).

4. A system (100, 200...,700) according to claim 3, wherein the control unit (120, 220..., 720) comprises a current sensor and a plurality of current switches.

5. A system (100, 200...,700) according to any of claims 1 to 4, wherein the plurality of control unit contacts (124, 224..., 724) is arranged to be retractable from an at rest position projecting from the control unit (120, 220..., 720) .

6. A control unit (120, 220..., 720) configured to receive and/or engage with a cartridge, comprising:
a plurality of control unit contacts (124, 224..., 724) for connecting to a cartridge (140, 220..., 720) having a plurality of cartridge contacts (144, 244..., 744), wherein at least one of the plurality of cartridge contacts (144, 244..., 744) is electrically coupled to at least one other of the plurality of cartridge contacts within the cartridge (144, 244..., 744) to form at least one pair of contacts, wherein the plurality of control unit contacts comprises at least four contacts and wherein at least two of the plurality of control unit contacts (124, 224..., 724) are power inputs
the control unit (120, 220..., 720) further comprising:
a power source; and
a controller for controlling delivery of power from the power source;
wherein the controller is configured to selectively provide power to the power inputs of the plurality of control unit contacts (124, 224..., 724), and wherein the control unit (120, 220..., 720) identifies a cartridge type associated with the cartridge (140, 220..., 720) based on which of the plurality of control unit contacts (124, 224..., 724) are in contact with which of the plurality of cartridge contacts (144, 244..., 744) and which of the at least one pair of contacts are determined to have a current flowing when the cartridge (140, 220..., 720) is coupled to the control unit (120, 220..., 720) for use.

7. A control unit (120, 220..., 720) according to claim 6, further comprising a current sensor.

8. A control unit (120, 220..., 720) according to claim 6 or 7, further comprising a plurality of current switches corresponding to the plurality of control unit contacts (124, 224..., 724).

9. A control unit (120, 220..., 720) according to any of claims 6 to 8, wherein the plurality of control unit contacts (124, 224..., 724) are arranged to be retractable from an at rest position projecting from the control unit (120, 220..., 720) .

10. A method of identifying a cartridge (140, 240, 340, 440, 540, 640, 740) using the cartridge identification system according to any one of claims 1 to 5, the method comprising:
connecting at least two contacts of the plurality of control unit contacts of the control unit and a corresponding at least two contacts of the a plurality of cartridge contacts of the cartridge of the cartridge identification system according to any one of claims 1 to 5, wherein at least one of the plurality of cartridge contacts is electrically coupled to at least one other of the plurality of cartridge contacts within the cartridge to form at least one pair of contacts; and
selectively providing power to the power inputs of the plurality of control unit contacts; and
identifying a cartridge type associated with the cartridge based on which of the plurality of control unit contacts are in contact with which of the plurality of cartridge contacts and which of the at least one pair of contacts are determined to have a current flowing.

11. A method of providing an aerosol using an aerosol provision system the method comprising:
identifying the cartridge (140, 240, 340, 440, 540, 640, 740) by using the cartridge identification system according to any one of claims 1 to 5;
selecting a heating profile based on the identified cartridge type and,
providing power to a heater (446, 546, 746) in accordance with the heating profile to provide an aerosol from an aerosol-generating material.

## Patentansprüche

1. Patronenidentifikationssystem (100, 200..., 700), das dazu konfiguriert ist, eine Patrone (140, 220..., 720) zum Enthalten eines aerosolerzeugenden Materials zu identifizieren, wobei das System Folgendes umfasst:
eine Patrone (140, 220 ..., 720), die einen Bereich zum Enthalten eines aerosolerzeugenden Materials umfasst und eine Vielzahl von Patronenkontakten (144, 244..., 744) aufweist;
eine Steuerungseinheit (120, 220..., 720), die dazu konfiguriert ist, die Patrone (140, 220..., 720) aufzunehmen und/oder mit ihr in Eingriff zu treten, wobei die Steuerungseinheit eine Vielzahl von Steuerungseinheitskontakten (124, 224..., 724) zum Verbinden mit der Patrone (140, 220..., 720) aufweist,
wobei mindestens einer aus der Vielzahl von Patronenkontakten (144, 244..., 744) elektrisch an mindestens einen anderen aus der Vielzahl von Patronenkontakten (144, 244..., 744) innerhalb der Patrone gekoppelt ist, um mindestens ein Kontaktpaar auszubilden,
wobei die Vielzahl von Steuerungseinheitskontakten mindestens vier Kontakte umfasst und wobei mindestens zwei aus der Vielzahl von Steuerungseinheitskontakten (124, 224..., 724) Leistungseingänge sind,
wobei die Steuerungseinheit (120, 220..., 720) ferner Folgendes umfasst:
eine Leistungsquelle (421); und
eine Steuerung zum Steuern der Leistungsabgabe von der Leistungsquelle;
wobei die Steuerung dazu konfiguriert ist, Leistung selektiv für die mindestens zwei Leistungseingänge aus der Vielzahl von Steuerungseinheitskontakten (124, 224..., 724) bereitzustellen, und die Steuerungseinheit (120, 220..., 720) einen Patronentyp, der der Patrone (140, 220..., 720) zugeordnet ist, basierend darauf identifiziert, welche aus der Vielzahl von Steuerungseinheitskontakten (124, 224..., 724) mit welchen aus der Vielzahl von Patronenkontakten (144, 244..., 744) in Kontakt sind und von welchen aus dem mindestens einen Kontaktpaar bestimmt ist, dass sie einen Stromfluss aufweisen, wenn die Patrone (140, 220..., 720) zur Verwendung an die Steuerungseinheit (120, 220..., 720) gekoppelt ist.

2. System (100, 200..., 700) nach Anspruch 1, wobei die Steuerungseinheit (120, 220..., 720) dazu angeordnet ist, bei Verwendung lösbar an die Patrone (140, 220..., 720) gekoppelt zu sein.

3. System (100, 200..., 700) nach einem der Ansprüche 1 bis 2, wobei die Steuerung die Leistungsabgabe von der Leistungsquelle zum Betreiben einer Heizung (446, 546, 746) steuert und
wobei Leistung von der Leistungsquelle über die Kontakte aus der Vielzahl von Steuerungseinheitskontakten (124, 224..., 724) in Kontakt mit Kontakten aus der Vielzahl von Patronenkontakten (144, 244..., 744) an die Patrone (140, 220..., 720) abgegeben wird.

4. System (100, 200..., 700) nach Anspruch 3, wobei die Steuerungseinheit (120, 220..., 720) einen Stromsensor und eine Vielzahl von Stromschaltern umfasst.

5. System (100, 200..., 700) nach einem der Ansprüche 1 bis 4, wobei die Vielzahl von Steuerungseinheitskontakten (124, 224..., 724) dazu angeordnet ist, aus einer Ruheposition, die von der Steuerungseinheit (120, 220..., 720) vorsteht, zurückziehbar zu sein.

6. Steuerungseinheit (120, 220..., 720), die dazu konfiguriert ist, eine Patrone aufzunehmen und/oder mit ihr in Eingriff zu treten, umfassend:
eine Vielzahl von Steuerungseinheitskontakten (124, 224..., 724) zum Verbinden mit einer Patrone (140, 220..., 720), die eine Vielzahl von Patronenkontakten (144, 244..., 744) aufweist, wobei mindestens einer aus der Vielzahl von Patronenkontakten (144, 244..., 744) elektrisch an mindestens einen anderen aus der Vielzahl von Patronenkontakten innerhalb der Patrone (144, 244..., 744) gekoppelt ist, um mindestens ein Kontaktpaar auszubilden, wobei die Vielzahl von Steuerungseinheitskontakten mindestens vier Kontakte umfasst und wobei mindestens zwei aus der Vielzahl von Steuerungseinheitskontakten (124, 224..., 724) Leistungseingänge sind,
wobei die Steuerungseinheit (120, 220..., 720) ferner Folgendes umfasst:
eine Leistungsquelle; und
eine Steuerung zum Steuern der Leistungsabgabe von der Leistungsquelle;
wobei die Steuerung dazu konfiguriert ist, Leistung selektiv für die Leistungseingänge aus der Vielzahl von Steuerungseinheitskontakten (124, 224..., 724) bereitzustellen, und wobei die Steuerungseinheit (120, 220..., 720) einen Patronentyp, der der Patrone (140, 220..., 720) zugeordnet ist, basierend darauf identifiziert, welche aus der Vielzahl von Steuerungseinheitskontakten (124, 224..., 724) mit welchen aus der Vielzahl von Patronenkontakten (144, 244..., 744) in Kontakt sind und von welchen aus dem mindestens einen Kontaktpaar bestimmt ist, dass sie einen Stromfluss aufweisen, wenn die Patrone (140, 220..., 720) zur Verwendung an die Steuerungseinheit (120, 220..., 720) gekoppelt ist.

7. Steuerungseinheit (120, 220..., 720) nach Anspruch 6, ferner umfassend einen Stromsensor.

8. Steuerungseinheit (120, 220..., 720) nach Anspruch 6 oder 7, ferner umfassend eine Vielzahl von Stromschaltern, die der Vielzahl von Steuerungseinheitskontakten (124, 224..., 724) entspricht.

9. Steuerungseinheit (120, 220..., 720) nach einem der Ansprüche 6 bis 8, wobei die Vielzahl von Steuerungseinheitskontakten (124, 224..., 724) dazu angeordnet ist, aus einer Ruheposition, die von der Steuerungseinheit (120, 220..., 720) vorsteht, zurückziehbar zu sein.

10. Verfahren zum Identifizieren einer Patrone (140, 240, 340, 440, 540, 640, 740) unter Verwendung des Patronenidentifikationssystems nach einem der Ansprüche 1 bis 5, wobei das Verfahren Folgendes umfasst:
Verbinden von mindestens zwei Kontakten aus der Vielzahl von Steuerungseinheitskontakten der Steuerungseinheit und einem entsprechenden mindestens zwei Kontakten aus der einer Vielzahl von Patronenkontakten der Patrone des Patronenidentifikationssystems nach einem der Ansprüche 1 bis 5, wobei mindestens einer aus der Vielzahl von Patronenkontakten elektrisch an mindestens einen anderen aus der Vielzahl von Patronenkontakten innerhalb der Patrone gekoppelt ist, um mindestens ein Kontaktpaar auszubilden; und
selektives Bereitstellen von Leistung für die Leistungseingänge der Vielzahl von Steuerungseinheitskontakten; und
Identifizieren eines Patronentyps, der der Patrone zugeordnet ist, basierend darauf, welche aus der Vielzahl von Steuerungseinheitskontakten mit welchen aus der Vielzahl von Patronenkontakten in Kontakt sind und von welchen aus dem mindestens einen Kontaktpaar bestimmt ist, dass sie einen Stromfluss aufweisen.

11. Verfahren zum Bereitstellen eines Aerosols unter Verwendung eines Aerosolbereitstellungssystems, wobei das Verfahren Folgendes umfasst:
Identifizieren der Patrone (140, 240, 340, 440, 540, 640, 740) unter Verwendung des Patronenidentifikationssystems nach einem der Ansprüche 1 bis 5;
Auswählen eines Heizprofils basierend auf dem identifizierten Patronentyp und
Bereitstellen von Leistung für eine Heizung (446, 546, 746) gemäß dem Heizprofil, um ein Aerosol aus einem aerosolerzeugenden Material bereitzustellen.

## Revendications

1. Système d'identification de cartouche (100, 200..., 700) conçu pour identifier une cartouche (140, 220..., 720) destinée à contenir un matériau de génération d'aérosol, le système comprenant :
une cartouche (140, 220 ..., 720) comprenant une zone destinée à contenir un matériau de génération d'aérosol et comportant une pluralité de contacts (144, 244..., 744) de cartouche ;
une unité de commande (120, 220..., 720) conçue pour recevoir et/ou venir en prise avec la cartouche (140, 220..., 720), l'unité de commande possédant une pluralité de contacts (124, 224..., 724) d'unité de commande en vue d'une connexion à la cartouche (140, 220..., 720), au moins l'un de la pluralité de contacts (144, 244..., 744) de cartouche étant couplé électriquement à au moins un autre de la pluralité de contacts (144, 244..., 744) de cartouche à l'intérieur de la cartouche pour former au moins une paire de contacts,
ladite pluralité de contacts d'unité de commande comprenant au moins quatre contacts, et au moins deux de la pluralité de contacts (124, 224..., 724) d'unité de commande étant des entrées d'énergie, ladite unité de commande (120, 220..., 720) comprenant en outre :
une source d'énergie (421) ; et
un dispositif de commande pour commander la distribution d'énergie à partir de la source d'énergie ;
ledit dispositif de commande étant conçu pour fournir sélectivement une énergie aux au moins deux entrées d'énergie de la pluralité de contacts (124, 224..., 724) d'unité de commande, et ladite unité de commande (120, 220..., 720) identifiant un type de cartouche associé à la cartouche (140, 220..., 720) sur la base de quels contacts de la pluralité de contacts (124, 224..., 724) d'unité de commande sont en contact avec quels contacts de la pluralité de contacts (144, 244..., 744) de cartouche et quels contacts de la au moins une paire de contacts sont déterminés comme possédant un courant circulant lorsque la cartouche (140, 220..., 720) est couplée à l'unité de commande (120, 220..., 720) lors de l'utilisation.

2. Système (100, 200..., 700) selon la revendication 1, ladite unité de commande (120, 220..., 720) étant agencée pour être couplée de manière libérable à la cartouche (140, 220..., 720) lors de l'utilisation.

3. Système (100, 200..., 700) selon l'une quelconque des revendications 1 à 2, ledit dispositif de commande commandant la distribution d'énergie à partir de la source d'énergie pour faire fonctionner un dispositif de chauffage (446, 546, 746), et
ladite énergie provenant de la source d'énergie étant distribuée à la cartouche (140, 220..., 720) par l'intermédiaire des contacts de la pluralité de contacts (124, 224..., 724) d'unité de commande en contact avec les contacts de la pluralité de contacts (144, 244..., 744) de cartouche.

4. Système (100, 200..., 700) selon la revendication 3, ladite unité de commande (120, 220..., 720) comprenant un capteur de courant et une pluralité de commutateurs de courant.

5. Système (100, 200..., 700) selon l'une quelconque des revendications 1 à 4, ladite pluralité de contacts (124, 224..., 724) d'unité de commande étant agencée pour être rétractable à partir d'une position de repos faisant saillie à partir de l'unité de commande (120, 220..., 720).

6. Unité de commande (120, 220..., 720) conçue pour recevoir et/ou venir en prise avec une cartouche, comprenant :
une pluralité de contacts (124, 224..., 724) d'unité de commande destinés à être connectés à une cartouche (140, 220..., 720) possédant une pluralité de contacts (144, 244..., 744) de cartouche, au moins l'un de la pluralité de contacts (144, 244..., 744) de cartouche étant couplé électriquement à au moins un autre de la pluralité de contacts de cartouche à l'intérieur de la cartouche (144, 244..., 744) pour former au moins une paire de contacts, ladite pluralité de contacts d'unité de commande comprenant au moins quatre contacts et au moins deux de la pluralité de contacts (124, 224..., 724) d'unité de commande étant des entrées d'énergie
ladite unité de commande (120, 220..., 720) comprenant en outre :
une source d'énergie ; et
un dispositif de commande pour commander la distribution d'énergie à partir de la source d'énergie ;
ledit dispositif de commande étant conçu pour fournir sélectivement de l'énergie aux entrées d'énergie de la pluralité de contacts (124, 224..., 724) d'unité de commande, et ladite unité de commande (120, 220..., 720) identifiant un type de cartouche associé à la cartouche (140, 220..., 720) sur la base des contacts de la pluralité de contacts (124, 224..., 724) d'unité de commande qui sont en contact avec les contacts de la pluralité de contacts (144, 244..., 744) de cartouche et de quels contacts de la au moins une paire de contacts qui sont déterminés comme possédant un courant circulant lorsque la cartouche (140, 220..., 720) est couplée à l'unité de commande (120, 220..., 720) lors de l'utilisation.

7. Unité de commande (120, 220..., 720) selon la revendication 6, comprenant en outre un capteur de courant.

8. Unité de commande (120, 220..., 720) selon la revendication 6 ou 7, comprenant en outre une pluralité de commutateurs de courant correspondant à la pluralité de contacts (124, 224..., 724) d'unité de commande.

9. Unité de commande (120, 220..., 720) selon l'une quelconque des revendications 6 à 8, ladite pluralité de contacts (124, 224..., 724) d'unité de commande étant agencés pour être rétractable à partir d'une position de repos faisant saillie à partir de l'unité de commande (120, 220..., 720).

10. Procédé d'identification d'une cartouche (140, 240, 340, 440, 540, 640, 740) à l'aide du système d'identification de cartouche selon l'une quelconque des revendications 1 à 5, le procédé comprenant :
la connexion d'au moins deux contacts de la pluralité de contacts d'unité de commande de l'unité de commande et au moins deux contacts correspondants de la pluralité de contacts de cartouche de la cartouche du système d'identification de cartouche selon l'une quelconque des revendications 1 à 5, au moins l'un de la pluralité de contacts de cartouche étant couplé électriquement à au moins un autre de la pluralité de contacts de cartouche à l'intérieur de la cartouche pour former au moins une paire de contacts ; et
la fourniture sélective de l'énergie aux entrées d'énergie de la pluralité de contacts d'unité de commande ; et
l'identification d'un type de cartouche associé à la cartouche sur la base de quels contacts de la pluralité de contacts d'unité de commande sont en contact avec quels contacts de la pluralité de contacts de cartouche et de quels contacts de la au moins une paire de contacts sont déterminés comme possédant un courant circulant.

11. Procédé de fourniture d'un aérosol à l'aide d'un système de fourniture d'aérosol, le procédé comprenant :
l'identification de la cartouche (140, 240, 340, 440, 540, 640, 740) à l'aide du système d'identification de cartouche selon l'une quelconque des revendications 1 à 5 ;
la sélection d'un profil de chauffage sur la base du type de cartouche identifié et,
la fourniture de l'énergie à un dispositif de chauffage (446, 546, 746) conformément au profil de chauffage pour fournir un aérosol à partir d'un matériau de génération d'aérosol.
